# FASCICULE DE BREVET EUROPEEN

(11) **EP 2 906 120 B1**
(45) Date de publication et mention de la délivrance du brevet: **06.11.2024**
(21) Numéro de dépôt: 13773294.7
(22) Date de dépôt: 13.09.2013
(51) Int. Cl.: A61B 5/22, A61B 5/00

(54) **DISPOSITIF DE MESURE DE L'EFFORT DE PINCEMENT ENTRE POUCE ET INDEX D'UNE PERSONNE**
VORRICHTUNG ZUR MESSUNG DER PRESSKRAFT ZWISCHEN DEM DAUMEN UND DEM ZEIGEFINGER EINER PERSON
DEVICE FOR MEASURING THE PINCH FORCE BETWEEN A PERSON'S THUMB AND INDEX FINGER

(30) Priorité: 14.09.2012 FR 1258673
(43) Date de publication de la demande: 19.08.2015
(73) Titulaire: Institut de Myologie, 75013 Paris (FR)
(72) Inventeur: HOGREL, Jean-Yves, 92120 Montrouge (FR); MORAUX, Amélie, 75012 Paris (FR)
(74) Mandataire: Gauchet, Fabien Roland
(86) Numéro de dépôt international: PCT/FR2013/052106
(87) Numéro de publication internationale: WO 2014/041315

(56) Documents cités:
- WO-A1-2011/044520
- DE-A1- 3 518 489
- US-A- 4 467 815
- US-A- 5 125 270
- US-A1- 2004 144 156
- US-A1- 2012 094 260
- US-B1- 7 096 731

## Description

### DOMAINE TECHNIQUE DE L'INVENTION

L'invention se rapporte au domaine de la mesure de l'effort (ou force) de pincement manuel entre le pouce et l'index d'une personne ; ce pincement est encore appelé 'pinch' en langue anglaise. Il s'agit donc de mesurer, afficher, traiter cet effort, notamment relativement à des personnes déficientes qui ne peuvent exercer que de très faibles efforts de pincement.

### ETAT DE LA TECHNIQUE ANTERIEURE

On connait déjà des dispositifs de mesure du pinch mais ils sont souvent dédiés à des personnes en bonne santé de sorte que de très faibles efforts ne peuvent être mesurés et traités avec efficacité et fiabilité.

On connait par exemple le document US 4 674 330 qui divulgue un dispositif qui comprend deux branches parallèles encastrées à l'une de leurs extrémités sur une pièce en forme d'étrier et dont les deux extrémités libres sont soumises à un effort de pression 'pinch' de la part de l'utilisateur qui exerce donc cet effort entre son pouce et son index au niveau des extrémités libres du dispositif. Des jauges de contrainte sont disposées dans une des branches de ce dispositif de sorte que l'effort mesuré peut être appliqué en tous points des extrémités. De plus ce dispositif présente la particularité de pouvoir modifier la distance entre les branches, afin de s'adapter à un deuxième type de mesure à savoir la force de préhension palmaire d'une personne. Ce dispositif présente donc la caractéristique d'une double fonctionnalité. La précision attendue et nécessaire pour la mesure du pinch d'une personne très déficiente n'est cependant pas obtenue avec un tel dispositif.

Le brevet US 2012/0137772 divulgue également un dispositif à double fonctionnalités à savoir la mesure d'une force palmaire et la mesure d'un effort entre deux doigts. Le principe de fonctionnement de ce dispositif est éloigné de celui présenté ci-avant. La structure associée à cette double fonctionnalité est tout aussi éloignée de celle de la présente invention ; en particulier aucune chambre de pression n'est nécessaire alors que ce dispositif antérieur repose sur une telle utilisation.

A titre d'arrière-plan technologique on connait un système de calibration de dispositif de mesure de l'effort de pincement, tel que présenté dans le brevet US 6 868 710 B1. Il s'agit ici de calibrer un dispositif de mesure connu en lui-même et représenté par exemple sur la figure 2 de ce document. Le dispositif de mesure est de type hydraulique et non pas mécanique.

Certains dispositifs pertinents dans le cadre de la présente invention sont également connus des documents US 5125270 A, DE 3518489 A1 et US 4467815.

Malheureusement les dispositifs connus ne permettent pas des mesures à la fois précises et pour de faibles valeurs d'efforts.

### EXPOSE DE L'INVENTION

L'invention vise à remédier aux inconvénients de l'état de la technique en vue notamment d'étudier les performances isométriques des muscles aussi bien de personnes très déficientes que des personnes en bonne santé, enfants comme adultes. Une fiabilité et une précision des mesures est opérée selon l'invention, de façon non encore obtenues à ce jour.

Pour ce faire est proposé selon un premier aspect de l'invention un dispositif selon la revendication 1.

Ladite première lame fait partie d'un cadre rigide et ladite deuxième lame est disposée dans le volume intérieur dudit cadre rigide. Ainsi, les deux lames sont reliées par le capteur selon une configuration particulière.

De façon préférée, le cadre rigide présente la forme extérieure d'un parallélogramme. Dans la suite de ce texte l'expression ' cadre rigide ' peut être assimilé à 'première partie du dispositif' dans la mesure où il renferme les éléments constitutifs de ladite première partie.

Cette disposition caractéristique du dispositif de mesure, outre les avantages déjà mentionnés ci-dessus, permet de s'affranchir de problèmes liés à la manière dont le sujet et/ou l'évaluateur tiennent le dispositif. On est ici assuré que, lors du fonctionnement, le dispositif est toujours tenu de façon adéquate parce que les surfaces découvertes, à savoir les extrémités des lames qui constituent les surfaces de contact fonctionnelles, sont les seules surfaces de contact possibles par le sujet.

Avantageusement, ledit cadre rigide présente une forme extérieure cylindrique, parallélépipédique ou sensiblement parallélépipédique.

Le capteur de mesure de ladite force est un capteur, de préférence à jauge de contrainte, intégré dans le volume intérieur dudit cadre rigide et agencé de façon à coopérer fonctionnellement avec la première et la deuxième lame. Bien entendu, d'autres capteurs connus de l'homme du métier peuvent être utilisés sans sortir du cadre de l'invention. Par exemple et de manière non limitative, un capteur piézoélectrique ou un capteur à compensation électromagnétique peuvent être utilisés.

Par exemple, le capteur de mesure de ladite force est un capteur à jauge de contrainte, comme ceux utilisés dans les balances de précision, fixé en suspension dans le volume intérieur dudit cadre rigide, et coopérant fonctionnellement avec la première et la deuxième lame.

Ainsi le cadre rigide constitue une protection pour le capteur lui-même ainsi que pour les autres composants sensibles du dispositif. Les perturbations et/ou dégradations des parties sensibles du dispositif sont de ce fait avantageusement diminuées. Le capteur en lui-même, d'une grande sensibilité, est protégé ce qui garantit une parfaite fiabilité et qualité des mesures. Ceci constitue un avantage considérable notamment lorsque des efforts très faibles doivent être mesurés.

**Par** ailleurs, le dispositif selon l'invention comprend un moyen de remise à zéro de la force mesurée, quelle que soit la position spatiale du dispositif. Cet aspect est intéressant pour s'affranchir des effets de la gravité auquel le capteur est sensible et qui peuvent biaiser la mesure dès lors que celle-ci est petite.

Avantageusement, le dispositif de mesure selon l'invention peut comprendre un moyen de sécurité destiné à empêcher le contact intempestif entre les deuxièmes extrémités longitudinales desdites lames, lorsque la force exercée est supérieure ou égale à un seuil donné S1. Le moyen de sécurité peut consister en une alarme ( visuelle, sonore ou autre ) ou en un élément mécanique.

De façon intéressante le dispositif selon l'invention comprend en outre un moyen de calibration et/ou d'étalonnage de ladite force exercée. Ce moyen peut prendre diverses formes sans sortir du cadre de l'invention.

Avantageusement le dispositif de mesure comprend en outre un moyen de transmission sans fil du signal relatif à ladite force de pincement enregistrée par ledit capteur de force.

Sans sortir du cadre de l'invention, le dispositif de mesure comprend en outre un moyen de communication à distance avec un appareil de traitement et/ou de stockage de données de type ordinateur.

De façon particulièrement avantageuse, le dispositif selon l'invention comprend en outre un moyen destiné au déclenchement à distance de l'acquisition du signal relatif à ladite force de pincement. Bien entendu cette option se révèle intéressante dans de nombreuses situations notamment lorsque l'environnement des mesures est difficile, mal approprié.

Le dispositif selon l'invention peut fonctionner grâce à une source d'énergie autonome de type batterie et/ou sur le secteur.

Selon un mode préféré de réalisation de l'invention, lesdits moyens d'affichage, de remise à zéro font partie d'un boitier indépendant du cadre rigide d'acquisition des données.

D'autres caractéristiques et avantages de l'invention ressortiront à la lecture de la description qui suit, en référence aux figures annexées, qui illustrent :
- la figure 1, une vue de côté d'un premier mode de réalisation de l'invention ;
- la figure 2, une vue de dessus du dispositif selon l'invention ; et
- la figure 3, une coupe longitudinale d'une partie du dispositif selon l'invention.

Pour plus de clarté, les éléments identiques ou similaires sont repérés par des signes de référence identiques sur l'ensemble des figures.

### DESCRIPTION DETAILLEE D'UN MODE DE REALISATION

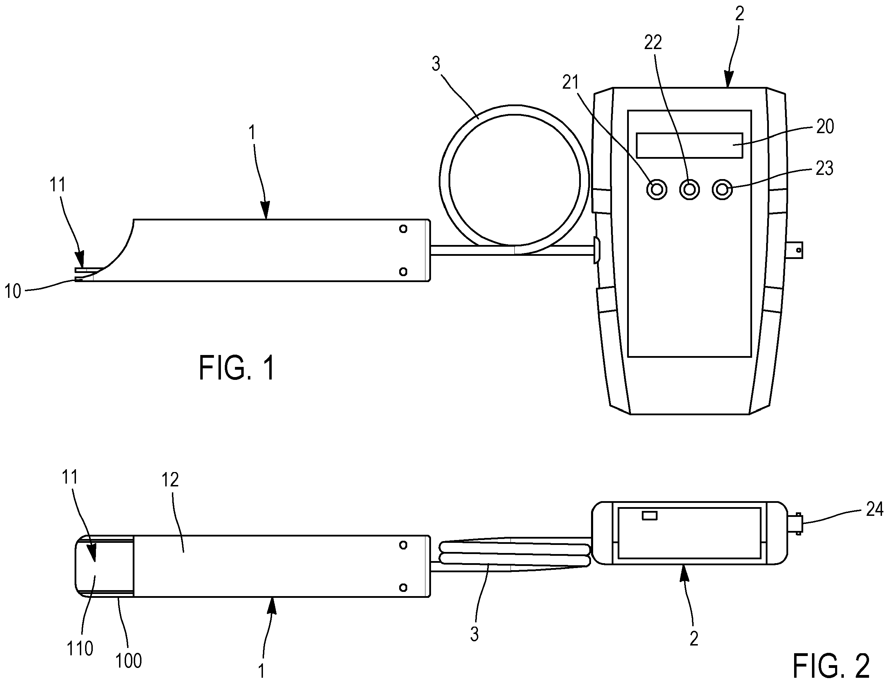

La figure 1 montre à titre illustratif les principaux éléments constitutifs de l'invention. On voit notamment une première partie 1 dite de mesures et une deuxième partie 2 de traitement, d'affichage et de transmission des mesures. Les deux parties sont de préférence reliées par un cordon de connexion 3.

La première partie comprend notamment une première lame 10 ainsi qu'une deuxième lame 11 ; les deux lames sont orientées parallèlement entre elles, à une distance donnée l'une de l'autre, par exemple environ sept millimètres. La première 10 et la deuxième 11 lame peuvent être considérées comme encastrées au niveau d'une première extrémité longitudinale et elles coopèrent avec un capteur de force 13 de telle sorte qu'un pincement au niveau des deuxièmes extrémités longitudinales et distales desdites lames, exercé perpendiculairement à leur plans principaux respectifs, soit directement répercuté sur ledit capteur de force 13.

La deuxième partie ou deuxième module 2 peut comprendre un dispositif d'affichage 20 de type connu en soi, par exemple à affichage digital ; il peut afficher un ou plusieurs paramètres de façon simultanée et/ou alternée, comme explicité ci-après. Au moins un premier contacteur 21 peut en outre être prévu afin de modifier l'état (arrêt ou fonctionnement) du dispositif ; au moins un deuxième contacteur-commutateur 22 peut être disposé au niveau du deuxième module 2 afin par exemple de déclencher une fonction de remise à zéro des mesures.

De plus un troisième contacteur 23 peut être prévu afin de déclencher à distance l'acquisition du signal donné par le capteur de force 13. Cette fonctionnalité est obtenue par tous moyens connus en eux-mêmes par l'homme du métier. Par exemple, il s'agit d'un caractère dans une chaîne d'information transmise par wifi. Bien entendu cette fonctionnalité est intéressante car il est ainsi possible de réaliser les mesures dans un premier lieu, et de transférer ces mesures en temps réel ( ou éventuellement en différé) par exemple vers un ordinateur permettant d'enregistrer et de traiter, en temps réel ou non, les signaux de mesure acquis par le capteur de force 13. Un signal wifi est ici visé, qui doit être synchronisé avec les autres signaux de transmission.

Sans sortir du cadre de l'invention, une prise BNC 24 peut être prévue pour la transmission filaire des données du capteur de force 13 vers un système d'acquisition. La figure 2 complète la figure 1 vis-à-vis de l'agencement extérieur d'un mode de réalisation de l'invention.

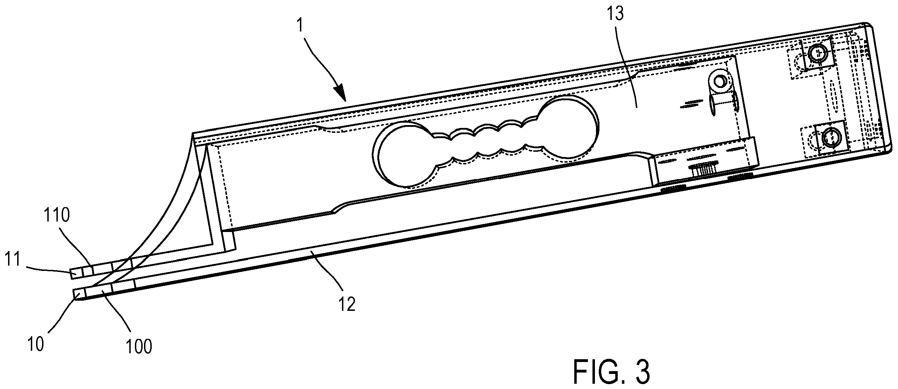

La figure 3 illustre schématiquement le montage fonctionnel de la première partie 1, ici de forme extérieure parallélépipédique puisque délimitée par un cadre rigide 12 de forme parallélépipédique. Le capteur de force 13 y est intégré de telle façon qu'une première surface soit fixée et en contact avec la première lame 10 tandis qu'une deuxième surface est fixée et en contact avec la deuxième lame 11. La première lame 10 est constituée par une face du cadre rigide 12 dont une partie 100 est destinée à être en contact avec un doigt de l'utilisateur ; la deuxième lame 11 comprend une partie fixée et en contact avec la deuxième surface fonctionnelle du capteur 13 qui est ici parallèle à la première surface. La deuxième lame 11 comprend une surface d'extrémité 110 destinée à être en contact avec l'un des doigts de l'utilisateur. Cette deuxième lame est donc en partie intégrée dans le cadre 12 et en partie découverte au niveau de sa surface d'extrémité fonctionnelle 110.

Les surfaces de contact 100 et 110 sont les surfaces distales respectivement des première 10 et deuxième 11 lames. A titre illustratif lesdites surfaces fonctionnelles de contact présentent une longueur d'environ 25 mm et une largeur proche ou supérieure.

Avantageusement les caractéristiques du capteur 13, par exemple un capteur de type AQ10 commercialisé par la société Scaime, permettent au système d'atteindre une précision de 10 grammes et une résolution de 1 gramme. En outre, il en résulte une stabilité des mesures dans le temps. Ainsi agencé, le capteur 13 permet une transmission fiable des valeurs de force, préférentiellement en temps réel ; les valeurs peuvent être transmises en différé sans sortir du cadre de l'invention. En outre ces valeurs peuvent être affichées en temps réel ou en différé sur le deuxième module 2 ou bien sur tout autre support tel que par exemple l'écran d'un ordinateur (non représenté). En fonction du paramétrage de la deuxième partie 2, la valeur maximale de l'effort enregistré, sur une période donnée, peut également être affichée. Cette valeur peut aussi être remise à zéro. Bien entendu d'autres traitements statistiques, évaluatifs et autres peuvent être effectués sur le signal donné par le capteur 13, sans sortir du cadre de l'invention. Les résultats de ces traitements peuvent être exploités pendant les mesures, afin par exemple d'optimiser les mesures. Ces résultats peuvent être traités ultérieurement notamment à des fins d'analyses diverses et variées comme le temps de montée en force, la stabilité de l'effort au cours d'efforts maximaux ou sous-maximaux soutenus, la fatigue lors de la répétition dynamique d'efforts maximaux...

Le mode opératoire préféré est le suivant : la première partie 1 du dispositif selon l'invention est tenue par l'évaluateur qui doit pincer les surfaces fonctionnelles 100 et 110 entre la deuxième phalange de son index et la pulpe de son pouce. Le poignet n'est pas fléchi et le pouce est tendu ; la première partie 1 du dispositif est placée dans l'alignement de l'avant-bras de l'utilisateur qui est confortablement posé sur une surface plane. La deuxième partie 2 du dispositif peut être placée à proximité ou à distance de la première partie 1.

## Revendications

1. Dispositif pour mesurer l'effort de pincement entre le pouce et l'index d'une personne, comprenant un cadre rigide (12) délimitant un volume intérieur, une première (10) et une deuxième (11) lames orientées parallèlement et à distance l'une de l'autre, maintenues solidaire au niveau de leurs premières extrémités longitudinales, un capteur (13) de mesure de la force de pincement exercée entre les deux lames au niveau de leurs deuxièmes extrémités longitudinales perpendiculairement à leurs plans principaux respectifs, le capteur (13) étant intégré dans le volume intérieur du cadre rigide (12), un moyen d'affichage (20) de ladite force de pincement, un moyen de mémorisation et/ou de traitement de ladite force de pincement **caractérisé en ce que** ladite première lame (10) est constituée par une face du cadre rigide (12) et **en ce que** ladite deuxième lame (11) est disposée dans le volume intérieur dudit cadre rigide.

2. Dispositif selon la revendication 1 **caractérisé en ce que** ledit cadre rigide (12) présente une forme extérieure cylindrique, parallélépipédique ou sensiblement parallélépipédique.

3. Dispositif selon la revendication 1 ou la revendication 2 **caractérisé en ce que** le capteur (13) de mesure de ladite force est un capteur, de préférence à jauge de contrainte, agencé de façon à coopérer fonctionnellement avec la première et la deuxième lame.

4. Dispositif selon l'une quelconque des revendications précédentes **caractérisé en ce qu'**il comprend un moyen (22) de remise à zéro de la force mesurée, quelle que soit la position spatiale du dispositif.

5. Dispositif selon l'une quelconque des revendications précédentes **caractérisé en ce qu'**il comprend un moyen de sécurité destiné à empêcher le contact intempestif entre les deuxièmes extrémités longitudinales (100, 110) desdites lames, lorsque la force exercée est supérieure ou égale à un seuil donné S1.

6. Dispositif selon l'une quelconque des revendications précédentes **caractérisé en ce qu'**il comprend en outre un moyen de calibration et/ou d'étalonnage de ladite force exercée.

7. Dispositif selon l'une quelconque des revendications précédentes **caractérisé en ce qu'**il comprend en outre un moyen de transmission sans fil du signal relatif à ladite force de pincement enregistrée par ledit capteur de force (13).

8. Dispositif selon l'une quelconque des revendications précédentes **caractérisé en ce qu'**il comprend en outre un moyen (24) destiné au déclenchement à distance de l'acquisition du signal relatif à ladite force de pincement.

9. Dispositif selon l'une quelconque des revendications précédentes **caractérisé en ce qu'**il comprend en outre un moyen de communication à distance avec un appareil de traitement et/ou de stockage de données de type ordinateur.

10. Dispositif selon l'une quelconque des revendications précédentes **caractérisé en ce qu'**il fonctionne grâce à une source d'énergie autonome de type batterie et/ou sur le secteur.

11. Dispositif selon l'une quelconque des revendications précédentes **caractérisé en ce que** lesdits moyens d'affichage, de remise à zéro font partie d'un boitier (2) indépendant du cadre rigide (12) d'acquisition des données.

## Patentansprüche

1. Vorrichtung zum Messen der Quetschkraft zwischen dem Daumen und dem Zeigefinger einer Person, umfassend einen starren Rahmen (12), der ein Innenvolumen begrenzt, eine erste (10) und eine zweite (11) Lamelle, die parallel zueinander und in einem Abstand voneinander ausgerichtet sind und an ihren ersten Längsenden zusammengehalten werden, einen Sensor (13) zum Messen der Quetschkraft, die zwischen den beiden Lamellen an ihren zweiten Längsenden senkrecht zu ihren jeweiligen Hauptebenen ausgeübt wird, wobei der Sensor (13) in das Innenvolumen des starren Rahmens (12) integriert ist, ein Mittel zum Anzeigen (20) der Quetschkraft und ein Mittel zum Speichern und/oder Verarbeiten der Quetschkraft, **dadurch gekennzeichnet, dass** die erste Lamelle (10) aus einer Fläche des starren Rahmens (12) besteht und die zweite Lamelle (11) im Innenvolumen des starren Rahmens angeordnet ist.

2. Vorrichtung nach Anspruch 1 , **dadurch gekennzeichnet, dass** der starre Rahmen (12) eine zylindrische, quaderförmige oder im Wesentlichen quaderförmige Außenform aufweist.

3. Vorrichtung nach Anspruch 1 oder Anspruch 2, **dadurch gekennzeichnet, dass** der Sensor (13) zum Messen der Kraft ein Sensor, vorzugsweise mit einem Dehnungsmessstreifen, ist, der derart angeordnet ist, dass er funktional mit der ersten und der zweiten Lamelle zusammenwirkt.

4. Vorrichtung nach einem der vorhergehenden Ansprüche, **dadurch gekennzeichnet, dass** sie ein Mittel (22) zum Zurücksetzen der gemessenen Kraft umfasst, unabhängig von der räumlichen Position der Vorrichtung.

5. Vorrichtung nach einem der vorhergehenden Ansprüche, **dadurch gekennzeichnet, dass** sie ein Sicherheitsmittel umfasst, das dazu bestimmt ist, den ungewollten Kontakt zwischen den zweiten Längsenden (100, 110) der Lamellen zu verhindern, wenn die ausgeübte Kraft größer oder gleich einem gegebenen Schwellenwert S1 ist.

6. Vorrichtung nach einem der vorhergehenden Ansprüche, **dadurch gekennzeichnet, dass** sie ferner ein Mittel zum Kalibrieren und/oder Eichen der ausgeübten Kraft umfasst.

7. Vorrichtung nach einem der vorhergehenden Ansprüche, **dadurch gekennzeichnet, dass** sie ferner ein Mittel zum drahtlosen Übertragen des Signals bezüglich der von dem Kraftsensor (13) gespeicherten Quetschkraft umfasst.

8. Vorrichtung nach einem der vorhergehenden Ansprüche, **dadurch gekennzeichnet, dass** sie ferner ein Mittel (24) umfasst, das zum Fernauslösen des Erfassens des Signals bezüglich der Quetschkraft bestimmt ist.

9. Vorrichtung nach einem der vorhergehenden Ansprüche, **dadurch gekennzeichnet, dass** sie ferner ein Mittel zur Fernkommunikation mit einem Gerät zur Datenverarbeitung und/oder -speicherung vom Typ Computer umfasst.

10. Vorrichtung nach einem der vorhergehenden Ansprüche, **dadurch gekennzeichnet, dass** sie mittels einer autonomen Energiequelle vom Typ Batterie und/oder mit Netzstrom betrieben wird.

11. Vorrichtung nach einem der vorhergehenden Ansprüche, **dadurch gekennzeichnet, dass** das Anzeigemittel und das Rücksetzmittel Teil eines Gehäuses (2) sind, das unabhängig von dem starren Rahmen (12) zur Datenerfassung ist.

## Claims

1. Device for measuring the pinch force between a person's thumb and index finger, comprising a rigid frame (12) defining an interior volume, a first (10) and a second (11) parallel blades oriented in parallel and at a distance from one another, maintained integral with their first longitudinal ends, a sensor (13) for measuring the pinch force exerted between the two blades on their second longitudinal ends perpendicularly to the respective main planes thereof, a means for displaying (20) said pinch force, a means for storing and/or processing said pinch force **characterised in that** said first blade (10) forms a face of the rigid frame (12) and **in that** said second blade (11) is arranged in the inside space of said rigid frame.

2. Device according to claim 1 **characterised in that** said rigid frame (12) has a cylindrical, parallelepiped or substantially parallelepiped exterior shape.

3. Device according to claim 1 or claim 2 **characterised in that** the sensor (13) for measuring said force is a sensor, more preferably with a strain gauge, integrated into the inside space of said rigid frame (12) and arranged in such a way as to engage functionally with the first and second blades.

4. Device as claimed in any preceding claim **characterised in that** it comprises a means (22) for clearing the force measured, regardless of the spatial position of the device.

5. Device as claimed in any preceding claim **characterised in that** it comprises a safety means intended to prevent untimely contact between the second longitudinal ends (100, 110) of said blades, when the force exerted is greater than or equal to a given threshold S1.

6. Device as claimed in any preceding claim **characterised in that** it further comprises a means for calibrating said force exerted.

7. Device as claimed in any preceding claim **characterised in that** it further comprises a means for the wireless transmission of the signal relating to said pinch force recorded by said force sensor (13).

8. Device as claimed in any preceding claim **characterised in that** it further comprises a means (24) intended for the remote triggering of the acquisition of the signal concerning said pinch force.

9. Device as claimed in any preceding claim **characterised in that** it further comprises a means of remotely communicating with a device for processing and/or storing data of the computer type.

10. Device as claimed in any preceding claim **characterised in that** it operates thanks to an autonomous power source of the battery type and/or from the mains.

11. Device as claimed in any preceding claim **characterised in that** said means for displaying, clearing are part of a case (2) independent of the rigid frame (12) for data acquisition.
